# EUROPÄISCHE PATENTSCHRIFT

(11) **EP 0 325 195 B1**
(45) Veröffentlichungstag und Bekanntmachung des Hinweises auf die Patenterteilung: **04.08.1993**
(21) Anmeldenummer: 89100693.4
(22) Anmeldetag: 17.01.1989
(51) Int. Cl.: A61L 27/00

(54) **Filzartiges Implantat**
Felt-like implant
Implant ayant l'aspect du feutre

(30) Priorität: 20.01.1988 DE 3801426
(43) Veröffentlichungstag der Anmeldung: 26.07.1989
(73) Patentinhaber: ETHICON INC., Somerville New Jersey 08876 (US)
(72) Erfinder: Schilder, Lothar, D-2000 Hamburg 50 (DE)
(74) Vertreter: UEXKÜLL & STOLBERG

(56) Entgegenhaltungen:
- EP-A- 0 176 895
- EP-A- 0 185 467
- GB-A- 2 181 438

## Beschreibung

Die Erfindung betrifft ein filzartiges Implantat aus resorbierbarem Werkstoff.

Derartige Implantate sind beispielsweise aus der US-PS 37 39 773 bekannt; sie bestehen aus flächigen oder velourartigen Produkten, die beispielsweise in mehreren Lagen oder in schwammartiger Struktur zur Behandlung von Brandwunden oder anderen Hautschäden und für andere Einsatzzwecke Verwendung finden. Aufgrund ihrer Porosität können sie Gewebeflüssigkeit aufnehmen und werden allmählich von Gewebe durchsetzt, das nach vollständiger Resorption des Implantates dessen Stütz- oder Haltefunktion übernimmt. Bei derartigem Flächengebilde können gemäß DE-OS 25 15 970 die an der Oberfläche liegenden Fasern mittels einer heißen Prägewalze in die Struktur hineingepreßt werden, um eine glattere Oberfläche zu ergeben, die sich bei Verwendung als Blutstill-Läppchen enger an die Wunde anschmiegt.

Diese textilartigen Flächengebilde sind jedoch wegen ihrer geringen Dicke und lappenartigen Beschaffenheit nur begrenzt einsetzbar.

Aus der DE-PS 36 19 197 sind kissenförmige Implantate bekannte, deren Außenhülle aus einem gewirkten Schlauch aus resorbierbaren Filamenten oder Fasern bestehen und mit Fäden, Filamenten, Flocken oder Schnitzeln aus einem resorbierbaren Material gefüllt sind; diese insbesondere zur Behandlung von Hernien geeigneten schlauchförmigen Polster haben eine Kompressibilität von mindestens 50% und werden ebenfalls zur Behandlung von Hernien eingesetzt.

Letztlich sind z.B. aus der US-PS 4 186 448 schaumstoffartige Implantate aus einem einheitlichen resorbierbaren Polyglykolsäureester bekannt; derartige schwammartige oder schaumstoffartige Implantate haben den Nachteil, daß sie noch Verunreinigungen aufgrund der zu Aufschäumung erforderlichen Bestandteile enthalten, die gewebeunverträglich sind; darüber hinaus haben derartige schaumstoffartige Implantate eine einheitliche Resorptionsdauer.

Der Erfindung liegt die Aufgabe zugrunde, ein Implantat vorzuschlagen, welches in jeder geometrischen Form herstellbar ist, welches schneidbar und einnähbar ist und welches in jeder beliebigen Konsistenz von weich bis fest erhältlich und dennoch hinreichend porös ist, um Gewebe einwachsen zu lassen, wobei vorzugsweise eine unterschiedliche Resorbierbarkeit erreicht wird.

Zur Lösung dieser Aufgabe wird ein filzartiges Implantat gemäß Hauptanspruch vorgeschlagen, wobei besonders bevorzugte Ausführungsformen in den Unteransprüchen aufgeführt sind.

Überraschenderweise hat sich gezeigt, daß ein filzartiges Implantat, das aus einem Gemisch verhältnismäßig kurzer Filamente oder Fasern von mindestens zwei verschiedenen resorbierbaren Materialien mit unterschiedlichen Schmelzpunkten besteht, und welches nach Erwärmen auf eine zwischen den Schmelzpunkten dieser verschiedenen resorbierbaren Materialien erwärmt und anschließend verformt worden ist, für zahlreiche Zwecke eingesetzt werden kann, in denen die bislang üblichen flächigen und textilartigen Gebilde oder kissenartige Implantatpolster nicht ausreichen.

Beispielsweise können filzartige Implantate in Pfropfenform bei der Marknagelung als Markraumstopfen eingesetzt werden, um ein weiteres Eindringen des den Stahlnagel fixierenden Kunstharzzementes in den weiteren Bereich des Röhrenknochens zu verhindern.

Die erfindungsgemäßen filzartigen Implantate sind auch als Ersatz der Dura mater encephali, also als Hirnhautersatz, geeignet, die als Schutzkapsel des Gehirns und Periost der Schädelinnenfläche dient. Bislang hat man für diesen Einsatzzweck nur aufbereitete lyophile Dura verwendet, die wegen etwaiger Infektionen insbesondere viröser Art ungeeignet ist.

Ein weiterer Einsatzzweck der erfindungsgemäßen filzartigen Implantate ist die Unterfütterung von Knochendefekten und als Zwischenlage bei Gefäßnähten, um ein Einschneiden des Nahtmaterials zu verhindern, sowie ferner beim Vernähen von anderen schwer vernähbaren Gewebebereichen, beispielsweise bei einer partiellen Leberresektion. Diese weichen bis festen Implantate sind insbesondere deswegen als Hirnhautersatz oder zur Unterfütterung von Knochendefekten und als Zwischenlage bei Gefäßnähten von Vorteil, weil die verschiedene Resorbierbarkeit der Filamente des Ausgangsfilzes ein besseres Einwachsen von Gewebe bei bleibender Retention von beispielsweise fixierten Teilen ermöglicht, wobei die weniger schnell resorbierbaren Bestandteile des ursprünglichen Filzes noch die erforderliche Festigkeit ermöglichen, bis das restliche Gewebe oder andere Körpersubstanz die Stelle der langsamer resorbierbaren Materialien eingenommen hat.

Resorbierbare Polymere mit guter physiologischer Verträglichkeit sind an sich bekannt. Typische Vertreter sind Poly-p-dioxanone, die unter der Bezeichnung "PDS" im Handel sind und chemisch gesehen aliphatische Polyester des Poly-p-dioxanons sind, sich zu Monofilamenten extrudieren lassen und bei 85 bis 95°C schmelzen; sie sind in "Ethicon OP Forum", Heft 108 (1981) und in J. Pediatr. Ophthalmol. 13, 360 - 364 (1976) beschrieben.

Zu einer anderen Gruppe resorbierbarer Materialien gehören die Polyglactine, die eine Copolymeres aus Glykolid und Lactiden sind und sich ebenfalls zu Fäden extrudieren lassen, die unter der Bezeichnung "Vicryl" (Wz) im Markt erhältlich und im einzelnen beispielsweise in "Ethicon OP Forum", Heft 96 (1978) und in J. Pediatr. Ophthalmol. 13, 360, 1976 beschrieben sind. Zu dieser Gruppe gehören auch Lactid-Glykolid-Blockcopolymerisate gemäß DE-OS 28 49 785. Diese Polyglactine haben je nach Polymerisationsgrad einen Schmelzpunkt von etwa 180 bis 200°C.

Je nach Art des resorbierbaren Materials und auch je nach Polymerisationsgrad ist die Resorbierbarkeit verschieden. Sie liegt beispielsweise bei den höher schmelzenden Polyglactinen in einem Bereich von 60 bis 80 Tagen und bei den niedriger schmelzenden Poly-p-dioxanonen in einem Bereich von 200 Tagen. Durch entsprechende Mischung der das filzartige Implantat bildenden Monofilamente oder Fasern kann man die Resorptionsdauer des gesamten filzartigen Implantats, also die Schnelligkeit, mit welcher das Implantat abgebaut werden kann, steuern.

Besonders bevorzugt ist eine Mischung von Fasern des Poly-p-dioxanons mit solchen des Polyglactins in einem Verhältnis von 5:1 bis 1:15 und insbesondere in einem Verhältnis von 1:3.

Die Faserlänge des Fasergemisches für filzartige Implantate beträgt 1 bis 20 mm und vorzugsweise 5 bis 12 mm, wobei der Durchmesser der Filamente oder Fasern 40 bis 200 dtex beträgt.

Dadurch, daß das Fasergemisch innerhalb der verschiedenen Schmelzpunkte der resorbierbaren Materialien erwärmt und danach verformt wird, ergibt sich eine Verfestigung der Fasern aus dem höher schmelzenden resorbierbaren Material durch aufschmelzendes Verkleben der Filamente oder Fasern aus dem resorbierbaren Material mit niedrigerem Schmelzpunkt, so daß ein nach wie vor poröses, wenngleich verhältnismäßig kompaktes filzartiges Gebilde erhalten wird. Die verhältnismäßig kurzen Filamente oder Fasern können ohne Schwierigkeiten bei der Erwärmung in einer entsprechenden Form miteinander zu einem filzartigen Körper, sei es zu einer Scheibe oder einem Pfropfen verpreßt werden.

### Beispiel

Es wurden Monofilamente aus einem Poly-(lactid-co-glykolid), nämlich Polyglactin 910, wie es unter der Bezeichnung "Vicryl" (Wz) erhältlich ist, und solche aus einem Poly-p-dioxanon, wie es unter der Bezeichnung PDS erhältlich ist, in einem Mischungsverhältnis von 3:1 durch Häckselung oder Vermahlung auf eine Fadenlänge von 5 bis 12 mm zerkleinert und nach intensivem Durchmischen auf 100°C erwärmt und in die gewünschte Form gepreßt. Hierbei wurde aufgrund des unterschiedlichen Schmelzpunktes der beiden Materialien eine Verklebung der Fäden aus höher schmelzendem Material erreicht und ein filzartiges Gebilde entsprechend der implantatgemäßen Form erhalten.

Ein derartiger Filzpfropf behielt seine Festigkeit aufgrund des Polyglactins mindestens 4 bis 5 Wochen, welcher auch nach Resorption dieses Materials noch weitere 2 bis 3 Wochen aufgrund des Poly-p-dioxanons eine ausreichende Formfestigkeit besaß.

## Patentansprüche

1. Filzartiges Implantat aus 1 bis 20 mm langen Filamenten oder Fasern aus resorbierbarem Werkstoff, **dadurch gekennzeichnet,** daß es aus einem Filamenten- oder Fasergemisch mindestens zweier verschiedener resorbierbarer Materialien mit unterschiedlichen Schmelzpunkten und unterschiedlicher Resorptionsdauer besteht, welches nach Erwärmen auf eine Temperatur, die oberhalb des Schmelzpunktes des resorbierbaren Materials mit dem niedrigsten Schmelzpunkt und unterhalb des Schmelzpunktes des anderen resorbierbaren Materials mit dem höchsten Schmelzpunkt liegt, zu einem filzartigen Körper verformt bzw. verpreßt worden ist.

2. Implantat nach Anspruch 1, dadurch gekennzeichnet, daß die Fasern aus einem Poly-p-dioxanon und aus Polyglactin in einem Mischungsverhältnis von 5:1 bis 1:15 bestehen.

3. Implantat nach Anspruch 2 dadurch gekennzeichnet, daß das Mischungsverhältnis der Fasern aus dem Poly-p-dioxanon und dem Polyglactin etwa 1:3 beträgt.

4. Implantat nach Anspruch 1 bis 3, dadurch gekennzeichnet, daß die Länge der Filamente oder Fasern 5 bis 12 mm beträgt.

5. Implantat nach Anspruch 1 bis 5, dadurch gekennzeichnet, daß die Filamente oder Fasern einen Durchmesser von 40 bis 20 dtex haben.

6. Filzartiges Implantat nach Anspruch 1, dadurch gekennzeichnet, daß das Fasergemisch bei einer Temperatur von 100 bis 100°C erwärmt und danach in die implantatgemäße Gestalt verformt worden ist.

## Claims

1. Feltlike implant comprising filaments or fibres of an absorbable material from 1 to 20 mm in length, characterised in that it consists of a filament or fibre mixture of at least two different absorbable materials having different melting points and different absorption times, shaped or moulded into a feltlike structure after heating to a temperature which is above the melting point of the absorbable material having the lowest melting point and above the melting point of the other absorbable material having the highest melting point.

2. Implant according to Claim 1, characterised in that the fibres consist of a poly-p-dioxanone and of polyglactin in a mixing ratio of from 5:1 to 1:15.

3. Implant according to Claim 2, characterised in that the mixing ratio of the fibres of poly-p-dioxanone and polyglactin is about 1:3.

4. Implant according to any of Claims 1 to 3, characterised in that the length of the filaments or fibres is from 5 to 12 mm.

5. Implant according to any of Claims 1 to 4, characterised in that the filaments or fibres have a diameter of from 40 to 200 dtex.

6. Feltlike implant according to Claim 1, characterised in that the fibre mixture has been heated at a temperature of from 100 to 110°C and then formed into the implant shape.

## Revendications

1. Implant du type feutre consistant en filaments ou fibres d'une longueur de 1 à 20 mm en matière résorbable, caractérisé en ce qu'il consite en un mélange de filaments ou de fibres d'au moins deux matières différentes résorbables, ayant des points de fusion différents et des durées de résorption différentes, qui, après chauffage à une température supérieure au point de fusion de la matière résorbable ayant le point de fusion le plus bas et inférieure au point de fusion de l'autre matière résorbable ayant le point de fusion le plus élevé, a été façonné ou comprimé en un corps analogue à un feutre.

2. Implant selon la revendication 1, caractérisé en ce que les fibres consistent en une poly-p-dioxannone et en polyglactine dans des proportions relatives de mélange de 5:1 à 1:15.

3. Implant selon la revendication 2, caractérisé en ce que les proportions relatives de mélange entre les fibres de la poly-p-dioxannone et de la polyglactine sont d'environ 1:3.

4. Implant selon l'une des revendications 1 à 3, caractérisé en ce que la longueur des filaments ou fibres est de 2 à 12 mm.

5. Implant selon les revendications 1 à 4, caractérisé en ce que les filaments ou fibres ont un diamètre de 40 à 200 dtex.

6. Implant de type feutre selon la revendication 1, caractérisé en ce que le mélange de fibres a été chauffé à une température de 100 à 110°C et soumis ensuite au façonnage en forme d'implant.
